# EUROPEAN PATENT SPECIFICATION

(11) **EP 3 930 667 B1**
(45) Date of publication and mention of the grant of the patent: **12.08.2026**
(21) Application number: 20705742.3
(22) Date of filing: 25.02.2020
(51) Int. Cl.: A61K 8/26, A61Q 11/02, A61K 8/19, A61Q 11/00

(54) **ORAL CARE COMPOSITION**
MUNDPFLEGEZUSAMMENSETZUNG
COMPOSITION D'HYGIÈNE BUCCALE

(30) Priority: 26.02.2019 EP 19159460
(43) Date of publication of application: 05.01.2022
(73) Proprietor: UNILEVER GLOBAL IP LIMITED, Wirral Merseyside CH62 4ZD (GB); Unilever IP Holdings B.V., 6708 WH Wageningen (NL)
(72) Inventor: BARILI, Matteo, 26841 Casalpusterlengo (IT); GUOLI, Angelica, 26841 Casalpusterlengo (IT); JOINER, Andrew, Wirral Merseyside CH63 3JW (GB); PHILPOTTS, Carole, Jane, Wirral Merseyside CH63 3JW (GB)
(74) Representative: Unilever Patent Group
(86) International application number: PCT/EP2020/054860
(87) International publication number: WO 2020/173917

(56) References cited:
- WO-A1-2017/068546
- WO-A1-2019/011512
- FR-A1- 2 458 282
- JP-A- S6 357 532
- US-A1- 2018 303 729
- DATABASE GNPD [online] MINTEL; 24 October 2017 (2017-10-24), ANONYMOUS: "Charcoal Whitening Toothpowder", XP055610113, retrieved from https://www.gnpd.com/sinatra/recordpage/5196787/ Database accession no. 5196787
- ANONYMOUS: "homemade tooth powder- remineralizing AND whitening! - almost exactly blog", 26 July 2013 (2013-07-26), XP055286028, Retrieved from the Internet <URL:https://almostexactlyblog.com/2013/07/26/homemade-tooth-powder-remineralizing-and-whitening/> [retrieved on 20160705]
- ANONYMOUS: "DIY natural healing activated charcoal toothpaste and tooth-powder recipes - Whitening too | Natural Frugal: Raising 6 kids", 10 May 2014 (2014-05-10), XP055286012, Retrieved from the Internet <URL:https://raising6kids.wordpress.com/2014/05/10/activated-charcoal-toothpaste/> [retrieved on 20160705]
- DATABASE GNPD [online] MINTEL; 24 October 2017 (2017-10-24), ANONYMOUS: "Charcoal Whitening Toothpowder", XP055610113, retrieved from www.gnpd.com Database accession no. 5196787

## Description

The present invention relates to an oral care composition that when used to brush the teeth mitigates staining.

White teeth are seen as cosmetically attractive, however the enamel of the teeth can stain, for instance when certain foods or drinks are consumed. Hence, there is a need for products that remove the staining of teeth.

WO2018060209 discloses compositions that whiten and mitigate tooth staining; the composition contains a whitening ingredient, a smectite clay and an acryl based polymer.

WO 2017/068546 describes a composition for oral hygiene comprising activated charcoal, ultra-ventilated green clay and white clay.

US 2018/0303729 describes a dentifrice composition comprising at least one phase containing activated carbon and an interference pigment.

FR 79/15133 describes a toothpaste containing 5 to 70 wt% charcoal having a D50 particle size of 5 to 210 microns as a tooth abrasive.

WO 2019/011512 describes a composition material containing a pigment having specified hue angle and a non-expanding bipolar 1:1 or 2:1:1 clay.

Almost Exactly Blog entitled "Homemade tooth powder - remineralizing and whitening!", retrieved from the Internet: URL:https://almostexactlyblog.com/2013/07/26/ homemade-tooth-powder-remineralizing-and-whitening, describes a toothpaste containing a clay and activated charcoal.

"DIY natural healing activated charcoal toothpaste and tooth-powder recipes - Whitening too", retrieved from the Internet: URL:https://raising6kids.wordpress.com/2014/05/10/ activated-charcoal-toothpaste, describes a toothpaste for whitening teeth containing a clay and activated charcoal.

Charcoal whitening toothpowder, sold by Superdrug, retrieved from www.gnpd.com, database accession no. 5196787, describes a toothpowder containing bentonite and charcoal.

There remains the need for a composition that when used to clean the teeth whitens and mitigates tooth staining.

### Description of the Invention

The present invention relates to an oral care toothpaste composition comprising
a) from 0.01 to 0.2 wt% of the total composition of charcoal; and
b) from 0.5 to 7 wt% of clay;
in which the weight ratio of charcoal to clay is from 1:100 to 5:100.

A further aspect of the invention is the above composition for use in a treatment to remove stains from the teeth.

### Detailed Description of the Invention

Compositions for use in the invention comprise charcoal. Preferably the charcoal has a total surface area (BET) of 900m²/g to 1500m²/g more preferably from 1100 m²/g to 1300. It is also preferred if the charcoal has a density from 250 kg/m³ to 370kg/m³, more preferably from 300 kg/m³ to 340 kg/m³. Preferably the charcoal has a D50 particle size from 2 microns to 40 microns, more preferably 5 to 30 microns.

The level of charcoal is from 0.01 to 0.2 wt% of the total composition, more preferably from 0.02 to 0.01 wt% from 0.03 to 0.08 wt% of the composition.

Compositions for use in the invention comprise clay, preferably this is a 1:1 layered silicate clay. Typically, the 1:1 layered silicate clay are non-swelling clays. 1:1 layered silicate clay includes kaolinite-serpentine group of clay. Kaolinite-serpentine group of clay includes subgroups of kaolinites and serpentines minerals. Serpentines are trioctahedral sheet minerals which has a tetrahedral sheet and an octahedral sheet having magnesium with minor amounts of aluminium and the species within this subgroup are preferably chrysotile, lizardite and antigorite.

Preferably the 1:1 layered silicate clay is a kaolinites clay. Kaolinites have a dioctahedral sheet and the species included within the kaolinites subgroup are kaolinite, dickite, nacrite and halloysite clay minerals, but not limited thereto. Kaolinite is particularly preferred in the disclosed invention.

Kaolinite, commonly known as kaolin clay, is a naturally occurring mineral. Kaolinite may be a calcined kaolin, highly purified calcined kaolin, colloidal kaolin, or hydrated kaolin, but not limited thereto. Preferred kaolinite have particle size distribution such that at least 98 wt percent of the particles have a particle size of 2 microns or less.

Preferably the kaolinite is a refined kaolin and further preferably the refined kaolin includes 38 wt percent Al2O3 and 45wt percent S1O2 and a maximum of 0.5 wt percent of Fe2C>3.

It is especially preferred if the clay is a hydrated aluminium silicate, with less than 0.025% of 53 micron or greater residues.

The weight ratio of charcoal to clay is from 1:100 to 5:100.

Compositions according to the invention may comprise a further whitening agent. The whitening agent preferably comprises a green and/or a blue pigment. In the context of the present invention a pigment is generally understood to be a shade/material which is insoluble in the relevant medium, at the relevant temperature. This is in contrast to dyes which are soluble. In the context of this invention, the "relevant medium" is human saliva, the liquid medium in which the composition is used, at the temperature of the oral cavity during brushing of the teeth, i.e. up to 37 Degrees C. As a reasonable approximation, the relevant medium may be considered to be water and the relevant temperature to be 25 Degrees C.

Preferably the blue pigment is Pigment Blue 15, more preferably Pigment Blue 15:1, 15:2, 15:3, 15:4, 15:5 or 15:6, most preferably 15:1. A preferred pigment is blue pigment is Phthalocyanine Blue Pigment, CI No. 74160, blue covarine.

The preferred Green pigment is Phthalocyanine Green, preferably Phthalocyanine Green CI-74260.

Preferably the composition is free of dye.

In one preferred embodiment of the invention the whitening system comprises a combination of green and blue pigment, the weight ratio of green pigment to blue pigment is greater than 1:2, preferably greater than 2:3 most preferably the weight ratio of green pigment to blue pigment is from 2:3 to 3:2.

Preferably the total level of pigment in the composition is from 0.01 wt% to 3 wt, more preferably from 0.02 to 2 wt%.

The composition may be a dual phase paste, with the whitening pigments present in one phase.

Compositions according to the invention preferably comprise a polymeric deposition aid.

Preferably the composition comprises acid anhydride polymers, particularly preferred are co-polymers of maleic anhydride with methyl vinylether, in which the anhydride moiety may be in a partially or fully hydrolysed or alcoholysed form. Preferred copolymers include Gantrez(R) polymers such as:
Gantrez S-95: molecular weight 216,000; free acid;
Gantrez S-96: molecular weight 700,000; free acid;
Gantrez S-97: molecular weight 1,500,000; free acid; and
Gantrez MS-955: molecular weight 1,060,000; calcium/sodium salt.

Particularly preferred co-polymers of maleic acid and methyl vinylether have a molecular weight of 1,000,000 or greater and an especially preferred material is Gantrez S-97.

Compositions of the invention are toothpastes.

Compositions according to the invention, , preferably comprise particulate abrasive materials such as silicas, aluminas, calcium carbonates, dicalciumphosphates, calcium pyrophosphates, hydroxyapatites, trimetaphosphates, insoluble hexametaphosphates and so on, including agglomerated particulate abrasive materials, usually in amounts between 3 and 60% by weight of the oral care composition.

Preferably the composition, comprises a silica based abrasive.

The preferred abrasive silicas used in the present invention is a silica with a low refractive index. It may be used as the sole abrasive silica, or in conjunction with a low level of other abrasive silicas, e.g. those according to EP 236070. The low refractive index silicas, used as abrasives in the present invention are preferably silicas with an apparent refractive index (R.I.) in the range of 1.41 - 1.47, preferably 1.435 - 1.445, preferably having a weight mean particle size of between 5 and 15 mm, a BET (nitrogen) surface area of between 10 and 100 m²/g and an oil absorption of about 70 - 150 cm³/100 g, but abrasive silicas with a lower apparent refractive index may also be used. Typical examples of suitable low refractive index abrasive silicas (e.g. having an R.I. of between 1.435 and 1.445) are Tixosil 63 and 73 ex Rhone Poulenc; Sident 10 ex Degussa; Zeodent 113 ex Zeofinn; Zeodent 124 ex Huber, Sorbosil AC 77 ex Crosfield Chemicals (having an R.I. of approximately 1.440). The amount of these silicas in the composition generally ranges from 5-60% by weight, usually 5-20% by weight.

The composition, preferably comprises an inorganic or a natural or synthetic thickener or gelling agent in proportions of about 0.10 to about 15% by weight depending on the material chosen. These proportions of thickeners in the dentifrice compositions of the present invention form an extrudable, shape-retaining product which can be squeezed from a tube onto a toothbrush and will not fall between the bristles of the brush but rather, will substantially maintain its shape thereon. Suitable thickeners or gelling agents useful in the practice of the present invention include inorganic thickening silicas such as amorphous silicas available from Huber Corporation under the trade designation Zeodent 165, Irish moss, iota-carrageenan, gum tragacanth, and polyvinylpyrrolidone. In the context of the present invention silica is particularly preferred.

The toothpaste composition will comprise further ingredients which are common in the art, such as:
antimicrobial agents, e.g. chlorhexidine, sanguinarine extract, metronidazole, quaternary ammonium compounds, such as cetylpyridinium chloride; bis-guanides, such as chlorhexidine digluconate, hexetidine, octenidine, alexidine; and halogenated bisphenolic compounds, such as 2,2' methylenebis-(4-chloro-6-bromophenol);
anti-inflammatory agents such as ibuprofen, flurbiprofen, aspirin, indomethacin etc.; anti-caries agents such as sodium- and stannous fluoride, aminefluorides, sodium monofluorophosphate, sodium trimeta phosphate and casein;
plaque buffers such as urea, calcium lactate, calcium glycerophosphate and strontium polyacrylates;
vitamins such as Vitamins A, C and E;
plant extracts;
desensitising agents, e.g. potassium citrate, potassium chloride, potassium tartrate, potassium bicarbonate, potassium oxalate, potassium nitrate and strontium salts; anti-calculus agents, e.g. alkali-metal pyrophosphates, hypophosphite-containing polymers, organic phosphonates and phosphocitrates etc.;
biomolecules, e.g. bacteriocins, antibodies, enzymes, etc.;
flavours, e.g. peppermint and spearmint oils;
proteinaceous materials such as collagen;
preservatives;
opacifying agents;
colouring agents;
pH-adjusting agents;
sweetening agents;
pharmaceutically acceptable carriers, e.g. starch, sucrose, water or water/alcohol systems etc.;
surfactants, such as anionic, nonionic, cationic and zwitterionic or amphoteric surfactants; Humectants such as glycerol, sorbitol, propyleneglycol, xylitol, lactitol etc.;
binders and thickeners such as sodium carboxymethyl-cellulose, hydroxyethyl cellulose (Natrosol^{®}), xanthan gum, gum arabic etc. as well as synthetic polymers such as polyacrylates and carboxyvinyl polymers such as Carbopol^{®};
polymeric compounds which can enhance the delivery of active ingredients such as antimicrobial agents can also be included;
buffers and salts to buffer the pH and ionic strength of the oral care composition; and
other optional ingredients that may be included are e.g. bleaching agents such as peroxy compounds e.g. potassium peroxydiphosphate, effervescing systems such as sodium bicarbonate/citric acid systems, colour change systems, and so on.

Example of the invention will now be illustrated by the following non-limiting examples:

### Examples

Examples were prepared using the base paste as in Table 1 and post dosing clay and charcoal to give test pastes summarised in Table 2.

**Table 1: Base Paste Formulation**

| **Ingredient** | **% w/w** |
|---|---|
| Water | 32.62 |
| Sorbitol | 45.00 |
| PEG | 2.00 |
| Sodium Fluoride | 0.32 |
| Sodium saccharin | 0.25 |
| Gantrez S-97 | 0.052 |
| Silica Thickening | 8.00 |
| Silica Abrasive | 9.00 |
| Cellulose gum | 0.75 |
| Sodium lauryl sulphate | 1.70 |
| Silica granules | 0.30 |

**Table 2: Test formulations summary**

| Formulation |
|---|
| Base Paste |
| Base Paste + 3% Clay |
| Base Paste + 3% Clay + 0.01% Charcoal |
| Base Paste + 3% Clay + 0.05% Charcoal |

The test formulations were evaluated for their stain removal properties using the Ferric-Tannate (FT) in vitro stain model. The method in outline is as follows. Hydroxyapatite (HAP) discs were cleaned and polished using P1200 silicon carbide paper. The baseline (initial clean) colour was measured using a chromameter. Tannic acid (0.5% w/w) and Ammonium iron sulphate hexahydrate (0.5% w/w) solutions were prepared and mixed in the ratio of 1:1 by volume to form the FT stain solution and used immediately. The FT stain solution was applied to the HAP discs using a small paintbrush and left to air dry. A further 3 to 4 coats of the stain were applied to each disc and allowed to dry. The colour of the discs was then remeasured (soiled).

The toothpastes were prepared as slurries in water 1:2 toothpaste. Sixteen HAP discs were used per treatment group. A stained HAP disc was placed into each well of a brushing machine and 10ml of the toothpaste slurry was applied. The HAP discs were then brushed for 1 minute with a flat trim toothbrush at a brushing speed of 150 cycles per min and load of 185g. The HAP discs were removed from the well, rinsed with water, gently dried and the colour was remeasured (cleaned). The brushing with toothpaste for a further 1 minute was conducted in the first experiment.

The % stain removal was calculated for each HAP disc using the equation below and the mean % stain removal determined. $% stain removal = \frac{L * \left(cleaned\right) - L * \left(soiled\right)}{L * \left(initial clean\right) - L * \left(soiled\right)} \times 100$

Table 3 and Table 4 demonstrate that a composition according to the invention comprising 3wt% clay and 0.05wt% charcoal is particularly effective at removing stains fromm the teeth compared with comparative Examples comprising 3wt% clay and 0.01wt% charcoal or clay alone .

**Table 3: Mean % Stain removal after 1 and 2 minutes brushing (Test 1).**

| **Formulation** | **1 minute** | | **2 minutes** | |
|---|---|---|---|---|
| | **Mean % Stain Removal** | **s.e.** | **Mean % Stain Removal** | **s.e.** |
| Base | 14.0^{A} | 4.6 | 42.0^{A} | 8.2 |
| Base + 3% Clay | 35.1^{B} | 7.4 | 52.4^{B} | 7.5 |
| Base + 3% Clay + 0.01% Charcoal | 30.9^{B} | 5.4 | 54.5^{B} | 7.7 |

| | | | | |
|---|---|---|---|---|
| Statistical differences indicated by different letters (p<0.05, ANOVA, Tukey-Kramer) | | | | |

**Table 4: Mean % Stain removal after 1 minute brushing (Test 2).**

| **Formulation** | **Mean % Stain Removal** | **s.e.** |
|---|---|---|
| Base | 13.55^{A} | 1.33 |
| Base + 3% Clay | 26.86^{B} | 0.98 |
| Base + 3% Clay + 0.05% Charcoal | 35.00^{C} | 0.91 |

| | | |
|---|---|---|
| Statistical differences indicated by different letters (p<0.05, ANOVA, Tukey-Kramer) | | |

The charcoal used had a total surface area (BET) of 1200m²/g; density 320 kg/m³, and D50 particle size of approximately 23microns.

## Claims

1. An oral care toothpaste composition comprising:
a) from 0.01 to 0.2 wt% of the total composition of charcoal; and
b) from 0.5 to 7 wt% of clay;
in which the weight ratio of charcoal to clay is from 1:100 to 5:100.

2. An oral care toothpaste composition according to claim 1 in which the charcoal has a D50 particle size from 2 microns to 40 microns.

3. An oral care toothpaste according to claim 1 or claim 2 in which the clay is kaolin.

4. An oral care toothpaste composition according to any preceding claim that further comprises a whitening agent.

5. An oral care toothpaste composition according to claim 4 wherein the whitening agent blue pigment comprises Phthalocyanine Blue Pigment.

6. An oral care toothpaste composition according to claim 4 wherein the whitening agent comprises a green pigment comprising Phthalocyanine Green.

7. An oral care toothpaste composition according to any preceding claim in which the composition comprises an acid anhydride polymer.

8. An oral care toothpaste composition according to any preceding claim that further comprises a silica based abrasive.

9. An oral care toothpaste composition according to any preceding claim that further comprises a surfactant.

10. An oral care toothpaste composition comprising:
a) from 0.01 to 0.2 wt% of the total composition of charcoal; and
b) from 0.5 to 7 wt% of clay; claim in which the weight ratio of charcoal to clay is from 1:100 to 5:100 for use in a treatment to remove stains from the teeth.

## Patentansprüche

1. Zahnpastazusammensetzung zur Mundpflege, umfassend:
a) 0,01 bis 0,2 Gew.-% der Gesamtzusammensetzung an Holzkohle; und
b) 0,5 bis 7 Gew.-% an Ton;
wobei das Gewichtsverhältnis von Holzkohle zu Ton zwischen 1:100 und 5:100 liegt.

2. Zahnpastazusammensetzung zur Mundpflege nach Anspruch 1, in der die Holzkohle eine D50-Partikelgröße von 2 Mikrometern bis 40 Mikrometern aufweist.

3. Zahnpastazusammensetzung zur Mundpflege nach Anspruch 1 oder Anspruch 2, in der der Ton Kaolin ist.

4. Zahnpastazusammensetzung zur Mundpflege nach einem vorhergehenden Anspruch, die ferner ein Aufhellungsmittel umfasst.

5. Zahnpastazusammensetzung zur Mundpflege nach Anspruch 4, in der das Aufhellungsmittel ein blaues Pigment ist, das Phthalocyaninblau-Pigment umfasst.

6. Zahnpastazusammensetzung zur Mundpflege nach Anspruch 4, in der das Aufhellungsmittel grünes Pigment ist, das Phthalocyaningrün umfasst.

7. Zahnpastazusammensetzung zur Mundpflege nach einem vorhergehenden Anspruch, in der die Zusammensetzung ein Säureanhydrid-Polymer umfasst.

8. Zahnpastazusammensetzung zur Mundpflege nach einem vorhergehenden Anspruch, die ferner ein Schleifmittel auf Siliciumdioxidbasis umfasst.

9. Zahnpastazusammensetzung zur Mundpflege nach einem vorhergehenden Anspruch, die ferner ein Tensid enthält.

10. Zahnpastazusammensetzung zur Mundpflege, die umfasst:
a) 0,01 bis 0,2 Gew.-% der Gesamtzusammensetzung an Holzkohle; und
b) 0,5 bis 7 Gew.-% an Ton;
wobei das Gewichtsverhältnis von Holzkohle zu Ton 1:100 bis 5:100 beträgt, zur Verwendung im Rahmen einer Behandlung zur Entfernung von Zahnverfärbungen.

## Revendications

1. Composition de dentifrice pour soins buccaux comprenant:
a) de 0,01 à 0,2 % en poids par rapport à la composition totale de charbon; et
b) de 0,5 à 7 % en poids d'argile;
dans laquelle le rapport pondéral du charbon à l'argile est de 1:100 à 5:100.

2. Composition de dentifrice pour soins buccaux selon la revendication 1 dans laquelle le charbon a une granulométrie D50 de 2 microns à 40 microns.

3. Dentifrice pour soins buccaux selon la revendication 1 ou la revendication 2 dans lequel l'argile est le kaolin.

4. Composition de dentifrice pour soins buccaux selon l'une quelconque des revendications précédentes qui comprend en outre un agent blanchissant.

5. Composition de dentifrice pour soins buccaux selon la revendication 4 dans laquelle l'agent blanchissant pigment bleu comprend du pigment bleu de phtalocyanine.

6. Composition de dentifrice pour soins buccaux selon la revendication 4 dans laquelle l'agent blanchissant comprend un pigment vert comprenant du vert de phtalocyanine.

7. Composition de dentifrice pour soins buccaux selon l'une quelconque des revendications précédentes dans laquelle la composition comprend un polymère d'anhydride d'acide.

8. Composition de dentifrice pour soins buccaux selon l'une quelconque des revendications précédentes qui comprend en outre un abrasif à base de silice.

9. Composition de dentifrice pour soins buccaux selon l'une quelconque des revendications précédentes qui comprend en outre un tensioactif.

10. Composition de dentifrice pour soins buccaux comprenant:
a) de 0,01 à 0,2 % en poids par rapport à la composition totale de charbon; et
b) de 0,5 à 7 % en poids d'argile;
dans laquelle le rapport pondéral du charbon à l'argile est de 1:100 à 5:100 destinée à être utilisée dans un traitement pour éliminer les taches des dents.
